Europäisches Patentamt

European Patent Office

Office européen des brevets

(19)

(11) EP 0 757 788 B1

(12) **EUROPÄISCHE PATENTSCHRIFT**

(45) Veröffentlichungstag und Bekanntmachung des Hinweises auf die Patenterteilung:
**28.11.2001   Patentblatt 2001/48**

(21) Anmeldenummer: **96902203.7**

(22) Anmeldetag: **26.02.1996**

(51) Int Cl.7: **G01N 19/08**, G01N 33/36, G01B 21/30

(86) Internationale Anmeldenummer:
**PCT/CH96/00058**

(87) Internationale Veröffentlichungsnummer:
**WO 96/27126 (06.09.1996 Gazette 1996/40)**

(54) **VERFAHREN UND VORRICHTUNG ZUR BESTIMMUNG VON STRUKTURFEHLERN AN LAUFENDEN TEXTILEN FLÄCHENGEBILDEN**

PROCESS AND DEVICE FOR DETECTING STRUCTURAL FAULTS OF MOVING FLAT TEXTILE MATERIALS

PROCEDE ET DISPOSITIF DE DETECTION DE DEFAUTS DE STRUCTURE DANS DES MATIERES TEXTILES PLATES EN MOUVEMENT

(84) Benannte Vertragsstaaten:
**AT CH DE FR GB IT LI**

(30) Priorität: **28.02.1995  CH 55895**

(43) Veröffentlichungstag der Anmeldung:
**12.02.1997   Patentblatt 1997/07**

(73) Patentinhaber: **Rhodia Industrial Yarns AG
6021 Emmenbrücke (CH)**

(72) Erfinder: **THALMANN, Markus
CH-6032 Emmen (CH)**

(74) Vertreter: **Herrmann, Peter Johannes, Dr.
Rhodia Filtec AG
Patentabteilung MP
6021 Emmenbrücke (CH)**

(56) Entgegenhaltungen:
EP-A- 0 372 690          WO-A-89/01152
DE-A- 2 058 101          DE-A- 2 627 239
DE-A- 3 721 488          US-A- 3 037 381
US-A- 5 287 742

• **TECHNISCHES MESSEN TM , Bd. 52, Nr. 7/8, Juli 1985 - August 1985, MUNCHEN DE, Seiten 288-291, XP002004542 SALJE ET AL: "Wegaufnehmer für hohe Tastgeschwindigkeiten"**

## Beschreibung

Die Erfindung betrifft ein Verfahren zur On-line-Bestimmung von Strukturfehlern an laufenden textilen Flächengebilden mittels eines starr eingespannten Berührungssensors sowie eine Vorrichtung zur Durchführung des Verfahrens.

Die Ermittlung von sichtbaren Strukturfehlern in textilen Flächengebilden werden in der Regel mittels optischen Methoden geprüft. So ist aus der EP-A-0562268 eine Vorrichtung bekannt, die unter anderem Kettfäden enthaltende Gewebe auf Fadenfehler mittels einer Beleuchtungseinrichtung, die in einem bestimmten Winkel gegenüber den Kettfäden verläuft, untersucht und mit einem lichtempfindlichen Element ausgestattet ist, welches ihre Signale an eine Auswerteelektronik weitergibt. Im Fall von optisch weniger deutlich ausgebildeten Fehlern funktioniert das Prüfverfahren nicht mehr.

Aufgabe der Erfindung ist es, ein Verfahren zu schaffen, das es ermöglicht, Strukturfehler, die quer zur Bewegungsrichtung des vorwärts bewegten Stoffes liegen, die ganze Breite betreffend, rasch zu bestimmen, d. h. zu erkennen, zu erfassen und direkt auszuwerten.

Eine weitere Aufgabe ist es, eine Messvorrichtung zur Verfügung zu stellen, welche die erkannten Strukturfehler misst und gleichzeitig auswertet.

Die Aufgabe wird erfindungsgemäss dadurch gelöst, dass die freie Länge des Berührungssensors von 5 bis 20 cm mit dem Flächengebilde einen Anstellwinkel α von 10-60 Grad bildet und die Bewegung des Berührungssensors als Messignal registriert und automatisch ausgewertet wird.

Da der Berührungssensor in der Halterung mit dem Messwertaufnehmer ein schwingungsfähiges System darstellt, sind auch die freie Länge des Berührungssensors, sein Querschnitt und die Anpresskraft, zusammen mit der Masse des Messwertaufnehmers von wesentlicher Bedeutung. Angeregt wird das System von der Rippelfrequenz, die sich aus der Struktur des Stoffes und der Vorschubgeschwindigkeit ergibt. Die Eigenfrequenz des Systems soll klein sein gegenüber der Rippelfrequenz. Eine freie Länge von 5 bis 20 cm, insbesondere 10 bis 20 cm, hat sich als zweckmässig erwiesen. Es ist auch zweckmässig, einen Anstellwinkel α von 10-60 Grad, insbesondere von 10-45 Grad, bevorzugt von 10-15 Grad vorzusehen. Ein Anstellwinkel α von weniger als 10 Grad hat den Nachteil, dass Fehler, die von einem einzigen Schussfaden herrühren, nicht erkannt werden, weil die benachbarten Schussfäden ein Absenken der Spitze der Berührungssonde in eine Vertiefung verhindern; ein Anstellwinkel α von mehr als 60 Grad bewirkt eine zu starke Abhängigkeit des Messignals von der Vorschubgeschwindigkeit des Stoffes.

Es ist zweckmässig, die Messung der Bewegung mittels eines Vibrationsaufnehmers durchzuführen. Dabei liefert die Intensität der Bewegung des Berührungssensors in Form einer Blattfeder die Information über die Stoffstruktur. Dabei ist es von Vorteil, dass der Vibrationsaufnehmer auch bei sehr kleinen Strukturfehlern noch auswertbare Signale liefern kann.

Es ist zweckmässig, in einer Varianten, die Messung der Bewegung mittels eines Wegaufnehmers durchzuführen. Während der Messung tastet der Berührungssensor mit der Abtastkante der Abrundung die Struktur des Stoffes ab und führt Bewegungen aus, die der Oberflächenstruktur des Stoffes entsprechen. Da der Messwertaufnehmer die Bewegung des Berührungssensors berührungslos abtastet, sind beträchtlich höhere Vorschubgeschwindigkeiten bzw. feinere Strukturen zu bestimmen möglich.

Es ist zweckmässig, in einer weiteren Varianten, die Messung der Bewegung mittels eines akustischen Aufnehmers durchzuführen. Wie beim Wegaufnehmer ist die Messung berührungslos. Es können daher höhere Vorschubgeschwindigkeiten gefahren und feinere Strukturfehler ermittelt werden. Zusätzlich liegt das Signal in einer Form vor, das den menschlichen Sinnen direkt zugänglich ist und somit vom Kontrollpersonal, beispielsweise über Kopfhörer, direkt erfasst werden kann.

Zur Durchführung des Verfahrens ist ein Berührungssensor vorgesehen, der als spatelförmige Blattfeder ausgebildet ist. Spatelförmig soll zum Ausdruck bringen, dass die Länge der Blattfeder ein Vielfaches ihrer Breite aufweist. Die Blattfeder besteht in ihrer einfachsten Ausführung aus gehärtetem Federbandstahl.

Es ist zweckmässig, die an einem Ende eingespannte Blattfeder an ihrem freien Ende mit einer konvexen Abrundung zu versehen. Die Abrundung hat den Vorteil, dass die parallel zum Kettfaden angeordnete Blattfeder sich beim Berühren der Textilbahn an ihren Kanten nicht verhaken kann. Ein weitere Vorteil wird darin gesehen, dass das eventuelle Verziehen des bewegten Stoffes während der Kontrolle, d. h. eine Nichteinhaltung des Winkels von 90 Grad zwischen Blattfeder und Schussfaden, nicht zu einer Einbusse an Empfindlichkeit führt.

Der Radius R der Abrundung liegt im Bereich von der halben Blattfederbreite bis zu R gleich 150 mm. Als besonders vorteilhaft hat sich ein Radius von 10 bis 100 mm, insbesondere 10 bis 40 mm erwiesen.

Das abgerundete Ende weist eine Abschrägung auf. Dabei bildet die Kante, die das Gewebe berührt, also auf der dem Gewebe zugewandten Seite, einen spitzen Winkel β. Der Winkel β wird festgelegt, abhängig von der Feinheit der zu messenden Struktur, aber auch durch die gewünschte Starrheit der Spitze. Die Spitze kann auch aus einem abriebfesten Material beispielsweise aus Keramik oder einem Korund bestehen.

Es ist von Vorteil, wenn der Winkel β 30 bis 90 Grad, insbesondere 50 bis 90 Grad, bevorzugt 60 bis 90 Grad, beträgt. Ein Winkel kleiner als 30 Grad hat den Nachteil, dass die Kante sich zu schnell abnutzt und weich ist; ein Winkel grösser als 90 Grad könnte bei bestimmten Werten für einen Anstellwinkel α zu einer Einbusse an Empfindlichkeit führen.

Es ist zweckmässig, den Messwertaufnehmer als Vibrationsaufnehmer auszubilden. Ein Vibrationsaufnehmer hat den Vorteil, dass auch bei sehr kleinen Strukturfehlern noch auswertbare Signale erhalten werden.

Es ist zweckmässig, in einer Varianten, den Messwertaufnehmer als Wegaufnehmer auszubilden. Ein Wegaufnehmer hat den Vorteil dass berührungslos abgetastet wird und so beträchtlich höhere Vorschubgeschwindigkeiten bzw. feinere Strukturen bestimmt werden können.

Es ist zweckmässig, in einer weiteren Varianten, den Messwertaufnehmer als akustischen Aufnehmer auszubilden. Ein akustischer Aufnehmer hat den Vorteil, dass die Messung berührungslos erfolgt. Es können daher höhere Vorschubgeschwindigkeiten gefahren und feinere Strukturfehler ermittelt werden. Zusätzlich liegt das Signal in einer Form vor, das den menschlichen Sinnen direkt zugänglich ist und somit vom Kontrollpersonal direkt erfasst werden kann.

Die Blattfeder liefert aber auch die Information über Grösse und Intensität der Bewegung über die Stoffstruktur. Somit kann die Strukturmessung in einer ersten Varianten auf eine Wegmessung reduziert werden. Dazu wird im Bereich des Blattfederendes der Messwertaufnehmer 4 in Form eines Abstandsensors derart am starren Teil der Halterung 3 befestigt, dass sich das Ende des Berührungssensors 2 im Bereich des Abstandsensors bewegt. Als Abstandsensoren kommen handelsübliche optische, magnetische, induktive oder kapazitive Wegaufnehmer in Frage.

Die Erfindung soll anhand einer Zeichnung näher beschrieben werden. Es zeigen:

Fig. 1     die erfindungsgemässe Vorrichtung in Seitenansicht

Fig. 2a     Draufsicht auf den Berührungssensor

Fig. 2b     Schnitt durch den Berührungssensors

Fig 3     Variante mit zwei Berührungssensoren

Fig. 4     das Schema einer Amplituden-Signalauswertung

Fig. 5     das Schema einer Frequenz-Signalauswertung

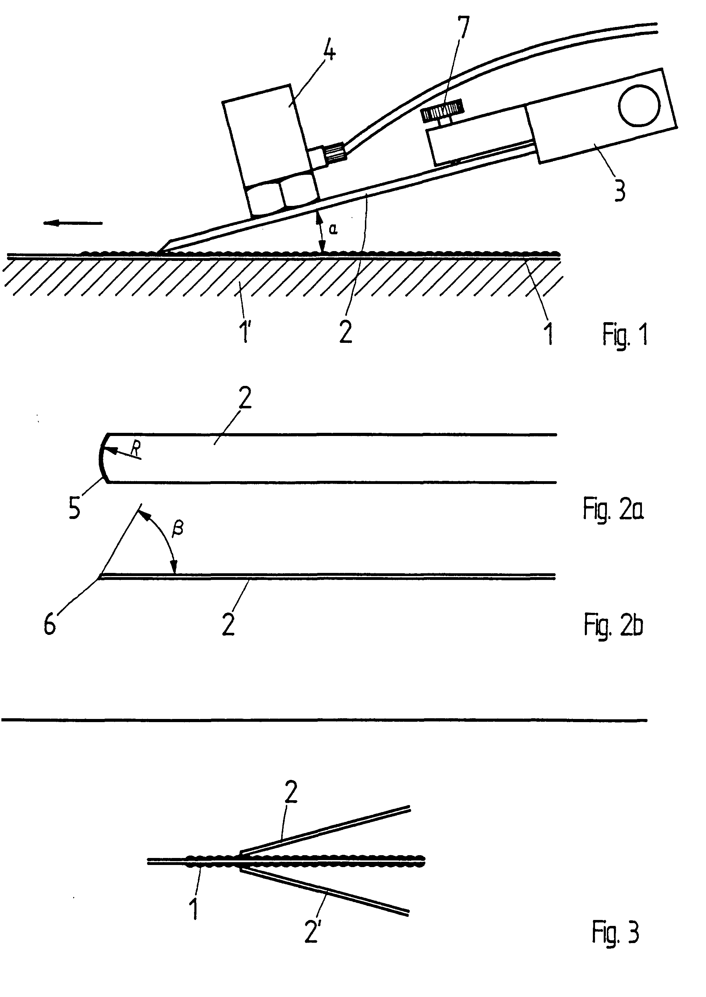

In Figur 1 ist mit dem Bezugszeichen 1 ein laufendes textiles Flächengebilde in Form einer zu prüfenden Stoffbahn gezeigt. Die Laufrichtung ist durch einen Pfeil angedeutet. Die Stoffbahn 1 liegt eben auf einer festen Unterlage 1'auf. Die Unterlage 1' besteht beispielsweise aus einer wenigstens 5 mm dicken Glasplatte. Ein Messkopf besteht in seinen wesentlichen Teilen aus einem Berührungssensor 2, einer Halterung 3 und einem Messwertaufnehmer 4. Der Messwertaufnehmer 4 ist lediglich in Form eines Vibrationsaufnehmers direkt mit dem Berührungssensor 2 verbunden. Bei den Varianten des Messaufnehmers 4 als Wegaufnehmer oder akustischer Aufnehmer sind diese zweckmässig oberhalb des Berührungssensors 2 angeordnet. In der Halterung 3 integriert befindet sich eine Stellschraube 7. Der Berührungssensor 2 bildet mit der laufenden Stoffbahn einen Winkel α.

In Fig. 2a ist der als Blattfeder ausgebildet Berührungssensor 2 dargestellt. Der Berührungssensor 2 weist an seiner Spitze eine konvexe Abrundung 5 mit einem Radius R auf.

In Fig. 2b ist eine Abschrägung 6 der Spitze des Berührungssensors 2 mit einem Winkel β ersichtlich.

Fig. 3 zeigt eine laufende Stoffbahn, die von einer oberen Seite mit dem Berührungssensor 2 und von der unteren Seite mit einem zweiten Berührungssensor 2' versehen ist. Diese Anordnung hat den Vorteil, dass das Nutzsignal doppelt so gross sein kann wie in der Ausführung mit einem Messkopf. Dazu müssen die Signale der beiden Aufnehmer phasenrichtig addiert werden. Die Phasenlage wird von der längsseitigen Versetzung der beiden Abtastkanten gegeneinander bestimmt und diese wiederum hängt von der Strukturgrösse (Schussfadentiter) des Gewebes ab.

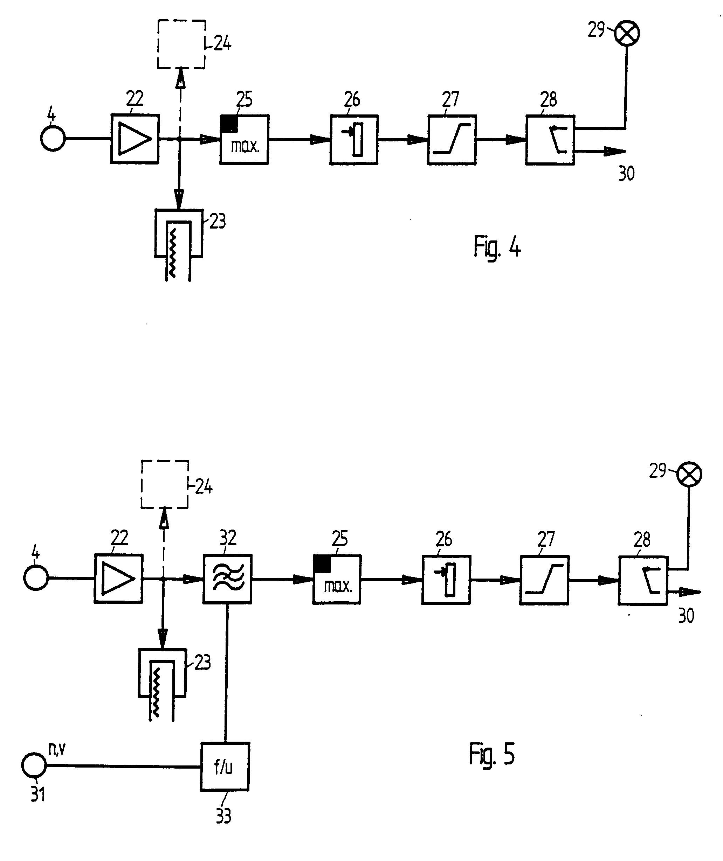

In Fig. 4 ist das Schema der Signalauswertung dargestellt. Das Messignal des Sensors 4, der die Bewegung des Berührungssensors 2 misst, wird in einem Verstärker 22 weiterverarbeitet. Das erhaltene Signal wird direkt mit einem Schnellschreiber 23 in bekannter Weise auf Papier geschrieben. Die regelmässige fehlerfreie Struktur des textilen Flächengebildes liefert für jeden Schussfaden einen Ausschlag und somit ein gleichmässig breites Band auf dem Schreiber. Die Fehlerstellen fallen als zu starke Signale gegenüber dem regelmässigen Signal sofort auf. Zur begleitenden visuellen Kontrolle kann ein Oszilloskop 24 beigestellt werden.

Zur weiteren Verarbeitung wird das Messignal einem Spitzenwertspeicher 25 zugeführt. Der Spitzenwertspeicher 25 speichert erforderlichenfalls das sehr kurze Fehlersignal, wenn es nur von einem betroffenen Faden herrührt. Der gespeicherte Spitzenwert wird beispielsweise auf einer Balkenanzeige 26 sichtbar gemacht. Mittels eines einstellbaren Grenzwertschalters 27 kann über einen Kontakt 28 ein Alarmsignal erhalten werden, das beispielsweise durch eine Alarmleuchte 29 das Bedienungspersonal aufmerksam macht oder per Fernsteuerung eine Maschine 30 ausschaltet.

In Fig. 5 ist die Signalauswertung gezeigt, die eine variable Vorschubgeschwindigkeit des Stoffes berücksichtigt. Die Frequenz des Messignals ergibt sich aus dem Produkt der Vorschubgeschwindigkeit und der Fadendichte.

$$f_{Regulär} = V_{Vorschub} \times \text{Schussfadendichte}$$

Schussfadendichte bedeutet Anzahl Schussfaden pro Längeneinheit.

Werden Unregelmässigkeiten, bedingt durch Strukturfehler im Gewebe, festgestellt, ist das an der Zunahme von andersfrequenten Anteilen im Signal ersichtlich. Um nur diese Störfrequenzen zu erfassen, wird die reguläre Frequenz $f_{Regulär}$ durch eine steuerbare Bandsperre 32 herausgefiltert. Das Steuersignal wird aus dem Sensor 31, der die Vorschubgeschwindigkeit misst, mittels Frequenz-Spannungswandler 33 gewonnen. Ein Strukturfehler erzeugt ein Ausgangssignal an der Bandsperre 32, was analog zur Beschreibung der Fig. 4 ausgewertet wird.

Im Betrieb sorgt die Halterung 3 dafür, dass der Berührungssensor 2 im richtigen Anstellwinkel $\alpha$ und der optimalen Anpresskraft den laufenden Stoff abtastet. Anpresskraft, Einspannlänge, Sensorengewicht und Geschwindigkeit der Stoffbahn müssen aufeinander abgestimmt sein. Die Anpresskraft kann, ohne dass die Halterung 3 verschoben wird mit der Stellschraube 7 frei eingestellt werden.

**Patentansprüche**

1. Verfahren zur On-line-Bestimmung von Strukturfehlern an laufenden textilen Flächengebilden (1) mittels eines starr eingespannten Berührungssensors (2), **dadurch gekennzeichnet, dass** die freie Länge des Berührungssensors (2) von 5 bis 20 cm mit dem Flächengebilde (1) einen Anstellwinkel $\alpha$ von 10-60 Grad bildet und die Bewegung des Berührungssensors (2) als Messignal registriert und automatisch ausgewertet wird.

2. Verfahren nach Anspruch 1, **dadurch gekennzeichnet, dass** die Messung der Bewegung mittels Vibrationsaufnehmer erfolgt.

3. Verfahren nach Anspruch 1, **dadurch gekennzeichnet, dass** die Messung der Bewegung mittels Wegaufnehmer erfolgt, der starr mit der Halterung (3) verbunden ist .

4. Verfahren nach Anspruch 1, **dadurch gekennzeichnet, dass** die Messung der Bewegung mittels akustischem Aufnehmer erfolgt, der starr mit der Halterung (3) verbunden ist .

5. Vorrichtung zur Durchführung des Verfahrens gemäß einem der vorhergehenden Ansprüche, bestehend aus einem Messkopf mit einem Berührungssensor (2), einer Halterung (3) und einem Messwertaufnehmer (4) zur Erfassung der Bewegung des Berührungssensors (2), **dadurch gekenn-**

**zeichnet, dass** der Berührungssensor (2) aus einer spatelförmigen Blattfeder, deren freie länge 5 bis 20 cm beträgt, besteht, die im Winkel von 90 Grad zur Schussfaden-Richtung angeordnet ist.

6. Vorrichtung nach Anspruch 5, **dadurch gekennzeichnet, dass** die Blattfeder an einem Ende eingespannt ist und an ihrem freien Ende eine konvexe Abrundung (5) aufweist.

7. Vorrichtung nach Anspruch 6, **dadurch gekennzeichnet, dass** der Radius R der Abrundung von der halben Blattfederbreite bis 150 mm beträgt.

8. Vorrichtung nach Anspruch 5, **dadurch gekennzeichnet, dass** die Blattfeder an einem Ende eingespannt ist und an ihrem freien Ende eine Abgeschrägung (6) aufweist, die einen spitzen Winkel $\beta$ bildet.

9. Vorrichtung nach Anspruch 5, **dadurch gekennzeichnet, dass** der Winkel $\beta$ 30 bis 90 Grad beträgt.

10. Vorrichtung nach Anspruch 5, **dadurch gekennzeichnet, dass** der Messwertaufnehmer (4) als Vibrationsaufnehmer ausgebildet ist.

11. Vorrichtung nach Anspruch 5, **dadurch gekennzeichnet, dass** der Messwertaufnehmer (4) als Wegaufnehmer ausgebildet ist.

12. Vorrichtung nach Anspruch 5, **dadurch gekennzeichnet, dass** der Messwertaufnehmer (4) als akustischer Aufnehmer ausgebildet ist.

**Claims**

1. Method for on-line determination of structural faults in running textile sheet materials (1) by means of a rigidly clamped tactile sensor (2), **characterized in that** the free length of the tactile sensor (2) of 5 to 20 cm forms a setting angle $\alpha$ of 10-60 degrees with the sheet material (1), and the movement of the tactile sensor (2) is recorded as measuring signal and automatically evaluated.

2. Method according to Claim 1, **characterized in that** the measurement of the movement is performed by means of vibration detectors.

3. Method according to Claim 1, **characterized in that** the measurement of the movement is performed by means of a position pickup which is rigidly connected to the holder (3).

4. Method according to Claim 1, **characterized in that** the measurement of the movement is performed by means of an acoustic pickup which is rigidly connected to the holder (3).

5. Device for carrying out the method in accordance with one of the preceding claims, comprising a measuring head with a tactile sensor (2), a holder (3) and a measuring sensor (4) for detecting the movement of the tactile sensor (2), **characterized in that** the tactile sensor (2) comprises a spatula-shaped leaf spring which has a free length of 5 to 20 cm and is arranged at an angle of 90 degrees to the weft thread direction.

6. Device according to Claim 5, **characterized in that** the leaf spring is clamped at one end and has a convex rounding (5) at its free end.

7. Device according to Claim 6, **characterized in that** the radius R of the rounding is from half the leaf spring width to 150 mm.

8. Device according to Claim 5, **characterized in that** the leaf spring is clamped at one end and has at its free end a bevel (6) which forms an acute angle β.

9. Device according to Claim 5, **characterized in that** the angle β is 30 to 90 degrees.

10. Device according to Claim 5, **characterized in that** the measuring sensor (4) is designed as a vibration detector.

11. Device according to Claim. 5, **characterized in that** the measuring sensor (4) is designed as a position pickup.

12. Device according to Claim 5, **characterized in that** the measuring sensor (4) is designed as an acoustic pickup.

## Revendications

1. Procédé pour la détection sur ligne de défauts de structure dans des matières textiles plates en mouvement (1), à l'aide d'un capteur de contact (2) fixé de manière rigide, **caractérisé en ce que** la longueur libre de 5 à 20 cm du capteur de contact (2) forme avec la matière textile plate (1) un angle d'incidence α de 10 - 60 degrés et que le mouvement du capteur de contact est enregistré comme signal de mesure et analysé de manière automatique.

2. Procédé selon la revendication 1, **caractérisé en ce que** la mesure du mouvement est réalisée à l'aide d'un capteur de vibration.

3. Procédé selon la revendication 1, **caractérisé en ce que** la mesure du mouvement est réalisée à l'aide d'un capteur de distance qui est fixé de manière rigide au support (3).

4. Procédé selon la revendication 1, **caractérisé en ce que** la mesure du mouvement est réalisée à l'aide d'un capteur acoustique qui est fixé de manière rigide au support (3).

5. Installation pour la mise en oeuvre du procédé selon une des revendications précédentes, composée d'une tête de mesure avec un capteur de contact (2), un support (3) et un enregistreur de données de mesure (4) pour la transcription du mouvement du capteur de contact (2), **caractérisée en ce que** le capteur de contact (2) se compose d'un ressort à lame en forme de spatule qui est positionné pour former un angle de 90 degrés avec l'axe des fils de trame.

6. Installation selon la revendication 5, **caractérisée en ce que** le ressort à lame est fixé à une extrémité et que l'autre extrémité montre une face arrondie convexe (5).

7. Installation selon la revendication 6, **caractérisée en ce que** le rayon R de l'arrondi de la demi-largeur du ressort à lame est inférieure à 150 mm.

8. Installation selon la revendication 5, **caractérisée en ce que** le ressort à lame est fixé à une extrémité et que l'autre extrémité montre une taille en biseau (6) qui forme un angle saillant β.

9. Installation selon la revendication 5, **caractérisée en ce que** l'angle β est compris entre 30 et 90 degrés.

10. Installation selon la revendication 5, **caractérisée en ce que** l'enregistreur de données de mesure (4) est configuré comme capteur de vibration.

11. Installation selon la revendication 5, **caractérisée en ce que** l'enregistreur de données de mesure (4) est configuré comme capteur de distance.

12. Installation selon la revendication 5, **caractérisée en ce que** l'enregistreur de données de mesure (4) est configuré comme capteur acoustique.

Fig. 1

Fig. 2a

Fig. 2b

Fig. 3

Fig. 4

Fig. 5